Europäisches Patentamt

European Patent Office

Office européen des brevets

(11) Publication number: **0 509 470 A2**

# EUROPEAN PATENT APPLICATION

(21) Application number: **92106483.8**

(22) Date of filing: **15.04.92**

(51) Int. Cl.⁵: **A61K 31/70,** //(A61K31/70, 31:52),(A61K31/70,31:44), (A61K31/70,31:415), (A61K31/70,31:505)

(30) Priority: **19.04.91 IT MI911097**

(43) Date of publication of application: **21.10.92 Bulletin 92/43**

(84) Designated Contracting States: **AT BE CH DE DK ES FR GB GR IT LI LU MC NL PT SE**

(71) Applicant: **REPLA CHEMICAL LTD. Hauptstrasse 27 FL-9490 Vaduz(LI)**

(72) Inventor: **La Colla, Paolo 5a Strada - Poggio dei Pini I-09012 Capoterra (Cagliari)(IT)** Inventor: **Marongiu, Maria Elena Via Dante, 99 I-09128 Cagliari(IT)** Inventor: **Pani, Alessandra Via Copernico, 3 I-09040 Settimo San Pietro (Cagliari)(IT)**

(74) Representative: **Gervasi, Gemma, Dr. et al NOTARBARTOLO & GERVASI Srl 33, Viale Bianca Maria I-20122 Milano(IT)**

(54) **Therapeutical compositions having anti-HIV activity consisting of 2',3'-didesoxyadenosine and substances inhibiting adenosine deaminase.**

(57) Therapeutical compositions, having enhanced anti-HIV activity consisting of 2', 3'-didesoxy-adenosine and of substances inhibiting the adenosine-deaminase selected from the group consisting of 9-(erythro-2-hydroxy-3-nonyl) adenine (EHNA), 3-deaza-EHNA, coformycin, desoxycoformycin, 1-deaza-EHNA, 1,3-dideaza-EHNA and 7-deaza-EHNA.

EP 0 509 470 A2

PRIOR ART

It is known that some retroviruses are the etiological agents of important human pathologies and particularly that the human immunodeficiency virus (HIV) classified in the types HIV-1 and HIV-2 constitutes the etiologic agent of the AIDS (acquired immunodeficiency syndrome).

Several substances have been recently described having as target inverse transcriptase, and therefore being able to selectively interfere with DNA synthesis of human retroviruses and in particular with that of HIV.

One of these substances, 3'-azido-2',3'-didesoxythymidine (AZT), has been used in AIDS therapy (M.A. Fischl et al., N. Engl.J. Med. 317, 185 (1987), F.A. Schmitt et al., N.Engl. J. Med. 319, 1573 (1988)). Anyway, a considerable percentage of patients affected by AIDS cannot tolerate the AZT therapy owing to the drug toxicity, while the improvement is only temporary in the rest of the patients. 2',3'-didesoxy-inosine (ddIno), represents at the moment a possible alternative to AZT, and its first clinical results demonstrate the absence of toxicity for the medulla ossium and sure effectiveness in reducing the HIV plasmatic levels (R. Yarchoan et al., Science 245, 412 (1989)).

The ddIno anti-HIV activity was found by carrying out an in vitro study on a series of 2',3' didesoxy-derivatives of purinic and pyrimidinic nucleotides, during which it was observed that the ddIno precursor, namely the 2',3'-didesoxy-adenosine (ddAdo), was able to inhibit the HIV multiplication in the same powerful and selective way as ddIno. That because both ddAdo and ddIno act through the formation of the same "active principle", the 2',3'-didesoxy-adenosine-triphosphate (ddATP), a selective inhibitor of the reverse transcriptase.

Studies conducted on ddAdo and ddIno metabolism in T lymphocytes have allowed to ascertain the conversion steps of each of the two analogues into ddATP (see Scheme 1).

In the case of ddAdo, it is firstly phosphorylated to 2',3'-didesoxy-adenosine-mono phosphate (ddAMP) by adenosine and desoxy-cytidino-kinase, and therefore it is further phosphorylated to ddATP by nucleotide-kinase. Alternatively, after its entry into the cells, ddAdo may be converted into ddIno by adenosine-deaminase (ADA).

In this case ddIno is firstly phosphorylated to 2',3'-didesoxy-inosine-monophosphate (ddIMP) by a 5'-cytoplasmatic-nucleotidase, then converted into ddAMP by the adenyl-succinate synthetase/lyase enzyme and finally phosphorylated to ddATP by nucleotide-kinase.

Since this metabolic route is the prevailing one in the human T lymphocytes, for therapeutic purposes it is preferable to use the ddIno, although its entry into the cells is 4-6 time less efficient than that of ddAdo (G. Ahluwalia et al., Biochem. Pharmacol. 36, 3797 (1987)). Finally it is known that the contemporaneous presence of a powerful ADA inhibitor, the 2'-desoxy-coformycin (2-dCF), does not promote an endocellular accumulation of ddATP starting from ddAdo (D.A. Cooney et al., Biochem. Pharmacol. 36, 1765 (1987)). That leads to the absence of enhancement of anti-HIV activity in vitro of ddAdo due to the presence of 2 dCF.

Summary

We have now unexpectedly found that some adenosine-deaminase inhibitors substances, i.e. coformycin (CF), desoxycoformycin (dCF), 9-(erythro-2-hydroxy-3-nonyl) adenine (EHNA) and some of its deazaderivatives enhance the anti-HIV activity in vitro of 2',3'-didesoxyadenosine (ddAdo) in a large range of concentrations which do not result toxic for the normal cells.

The present invention therefore refers to the use of such substances for the preparation of therapeutic compositions comprising ddAdo and said substances. The thus obtained compositions show an enhanced anti-HIV activity.

Detailed description of the invention

The characteristics and the advantages of the compositions consisting of 2',3' didesoxyadenosine (ddAdo) and of substances able to inhibit adenosine-deaminase (ADA) according to the present invention will be more pointed out in the following detailed description.

The activity of said compositions has been studied during in vitro tests in order to define the possibility of their utilization in AIDS therapy and it has been found that substances able to inhibit ADA and suitable for the preparation of these compositions are EHNA, 3-deaza-EHNA, CF, dCF, 1-deaza-EHNA, 1,3-dideaza-EHNA and 7-deaza-EHNA.

The cellular lines utilized in this study were:

H9, T lymphocytes, CD4 +, permissive for the HIV replication but partially resistant to its cytopathic effect;

- H9/IIIB, H9 lymphocytes chronically infected by HTLV/IIIB virus;
- MT4 and C8166, T lymphocytes lines, CD4 + permissive for HIV replication. In C8166 the virus induces a cytopathic effect which results in the formation of syncytia easily visible at the optical microscope.

All the lymphoid lines were cultured in RPMI 1640 containing 10 % of non decomplemented fetal calf serum (FCS), penicillin 100U/mL and streptomycin 100 $\mu$g/mL and incubated at 37 °C in 5% $CO_2$.

HIV virus was obtained by H9/IIIB cells surnatant taken at the end of their exponential growth.

The titre of several stock preparations was comprised between 2 and 5 x $10^5$ Cell Culture Infection Dose 50 (CCID50)/mL.

For the cytotoxicity evaluation, the cells were resuspended in a culture medium at a density of 1 x $10^5$/mL, were incubated in the absence or in the presence of various concentration of the compounds to be studied and their number was determined in Coulter after 96 hours of incubation at 37 °C.

For the evaluation of the anti-HIV activity the inhibition of infecting HIV production was determined as follows.

C8166 or MT4 cells sown at a density of 1 x $10^6$ cells/ml, were infected with a multiplicity of infection (m.o.i.) comprised between 0.05 and 0.02 CCID50/cell. After two hours of incubation at 37 °C and subsequent removal of the inoculum, the cells were washed three times and resuspended at a density of 1 x $10^5$ cells/ml. in the absence or in the presence of the compounds to be investigated.

After 4 days of incubation at 37 °C the presence of syncytia was evaluated at the optical microscope and the quantity of infecting HIV present in the surnatant of the cultures was determined by end point titration.

HIV titration was carried out in C8166 cells sown at a density of 1 x $10^5$ cells/mL/well, by the limit dilution method (dilution ratio 1:2, every dilution being sown in quadruplicate).

After four days of incubation, the syncytia presence was evaluated at the microscope and the viral titre was calculated by the Reed and Muench method.

The results of the in vitro evaluation of toxicity of ddAdo, ddIno, Coformycin (CF), EHNA and its deaza derivatives in C8166 and MT4 cells are reported in table 1.

From this table one can note that ddAdo and ddIno result the substance having the lowest cytotoxicity. Their maxima non cytotoxic doses (MNTD) are in fact equal or higher than 1 mM both for C8166 and MT4 cells.

Among the ADA inhibitors, CF is cytotoxic at 250 $\mu$M while EHNA and its deaza-derivatives have a MNTD comprised between 5 and 20 $\mu$M and a TD50 comprised between 15 and 75 $\mu$M.

Table 1. Toxicity of ddAdo, ddIno, CF, EHNA and its deaza derivatives in C8166 and MT4 cells:

| COMPOUND | C8166 | | (μM) | MT4 | |
|---|---|---|---|---|---|
| | (a)MNTD | (b)TD50 | | MNTD | TD50 |
| ddAdo | >1000 | >1000 | | 1000 | >1000 |
| ddIno | >1000 | >1000 | | >1000 | >1000 |
| CF | > 250 | > 250 | | > 250 | > 250 |
| EHNA | 15 | 25 | | 10 | 35 |
| 1-deaza-EHNA | ND | ND | | 20 | 75 |
| 3-deaza-EHNA | ND | ND | | 10 | 30 |
| 7-deaza-EHNA | ND | ND | | 5 | 50 |
| 1,3-dideaza-EHNA | ND | ND | | 5 | 15 |

(a) MNTD (maximum Non Toxic Dose): concentration of a compound able to allow three subsequent cycles of cellular multiplication.

(b) TD50 (Toxic Dose 50): concentration of a compound necessary to reduce by 50% the cells number under experimental conditions allowing the untreated controls to grow exponentially for three subsequent cycles.

The effect of ddAdo, ddIno, CF, EHNA and its deaza-derivatives on the HIV-1 multiplication in C8166 and MT4 cells is shown in tab. 2 which reports the concentrations of various inhibitors, necessary to reduce by 90 % the multiplication of infecting HIV in a quantity reduction test of infecting viruses.

As it can be noted in said table, ddAdo and ddIno are equivalent in the antiviral action independently on the type of cells used.

On the contrary, CF, EHNA, and its deaza-derivatives are not able to prevent the HIV multiplication also at concentration equivalent to the corresponding MNTD.

The only exception is represented by 1-deaza EHNA, that at 20 $\mu$M reduce by 90 % HIV multiplication.

Table 2

| Effect of ddAdo, CF, EHNA and its deaza-derivatives on the HIV-1 multiplication in C8166 and MT4 cells. | | |
|---|---|---|
| COMPOUND | (a)ED90 ($\mu$M) | |
| | C8166 | MT4 |
| ddAdo | 25 | 10 |
| ddIno | 28 | 11 |
| Coformycin | > 250 | > 250 |
| EHNA | > 15 | > 10 |
| 1-deaza-EHNA | ND | 20 |
| 3-deaza-EHNA | ND | > 10 |
| 7-deaza-EHNA | ND | > 5 |
| 1,3-dideaza-EHNA | ND | > 5 |

(a) ED90 (Effective Dose 90): compound concentration necessary to reduce by 90 % HIV multiplication. The virus titre in untreated controls was $2.6 \times 10^5$ (C8166) and $5.6 \times 10^5$ (MT4) CCID50/mL.

The ddAdo/EHNA association effect on the HIV-1 multiplication is shown in Tab. 3 wherein it is possible to notice that EHNA enhance the ddAdo anti-HIV-1 activity.

The ED90, ED99 and ED99.9 of ddAdo, used alone or in combination with the indicated EHNA concentrations, are reported in Tab. 3.

As one can observe, EHNA enhances the ddAdo anti-HIV activity up to concentration 250 times lower than its MNTD.

In association with EHNA, ddAdo inhibits by one, two or three magnitude orders the HIV multiplication at doses from 2 to 8 times lower than those necessary to produce the same inhibition degree as that obtained when ddAdo is used alone.

Results similar to those obtained for C8166 cells were also obtained for MT4 cells.

Table 3

| Anti-HIV-1 effect of ddAdo, alone or in association with EHNA. | | | |
|---|---|---|---|
| EHNA ($\mu$M) | ddAdo ($\mu$M) | | |
| | (a)ED90 | ED99 | ED99.9 |
| Absent | 25.0 | 39.0 | 61.0 |
| 15 | 3.0 | 7.5 | 17.5 |
| 5 | 3.5 | 10.0 | 23.5 |
| 1.6 | 5.0 | 11.5 | 25.5 |
| 0.5 | 6.5 | 14.5 | 34.5 |
| 0.2 | 7.5 | 18.0 | 42.5 |
| 0.06 | 10.5 | 30.5 | 52.0 |

(a)ED90, ED99, ED99.9 (Effective dose 90, 99, 99.9): concentration of ddAdo alone or in association, necessary to reduce by 90, 99 or 99.9 % HIV-1 multiplication in C8166 cells.

The virus titre in untreated controls was $2.7 \times 10^5$ CCID50/mL.

The ddAdo/CF association effect on the HIV-1 multiplication in MT4 cells is shown in Tab. 4 wherein it is possible to observe that CF enhances the ddAdo anti-HIV-1 activity.

The ED90, ED99 and ED99.9 of ddAdo, used alone or in association with the indicated concentrations of CF, are reported in Tab. 4. CF enhances ddAdo anti-HIV activity up to concentrations more than 12500 times lower than its MNTD.

In association with CF, ddAdo reduces by one, two or three orders of magnitude the HIV multiplication at concentrations from 2 to 11 times lower than those necessary to produce the same inhibition degree as that obtained when it is used alone.

Table 4

| Anti-HIV-1 effect of ddAdo, alone or in association with Coformycin. | | | |
|---|---|---|---|
| Coformycin (CF) | ddAdo ($\mu$M) | | |
| | (a)ED90 | ED99 | ED99.9 |
| Absent | 11.0 | 19.0 | 32.0 |
| 45 | 1.0 | 2.5 | 7.0 |
| 15 | 1.5 | 3.5 | 7.5 |
| 5 | 2.0 | 4.0 | 7.5 |
| 1.6 | 2.5 | 4.5 | 7.5 |
| 0.5 | 3.6 | 5.0 | 8.0 |
| 0.2 | 3.0 | 5.0 | 8.0 |
| 0.06 | 3.5 | 7.1 | 12.5 |
| 0.02 | 4.0 | 7.6 | 13.7 |

(a)ED90, ED99, Ed99.9 (Effective dose 90, 99, 99.9): concentration of ddAdo, alone or in association, necessary to reduce by 90, 99, or 99.9 % the HIV-1 multiplication in MT4 cells.

The virus titre in untreated controls was $3.0 \times 10^5$ CCID50/mL.

It was also determined the effect of the ddAdo/desoxycoformycin (dCF) association on the HIV-1 multiplication in MT4 cells.

Before the association tests, the dCF toxicity and anti-HIV activity were evaluated, both in C8166 cells and in MT4 cells.

In both cellular lines dCF resulted as non toxic and without any anti-HIV-1 activity at concentrations up to 500 $\mu$M.

Table 5 reports the results of the anti-HIV-1 effect in different concentrations of ddAdo used alone or in association with the indicated dCF concentrations.

dCF enhances the ddAdo anti-HIV activity up to concentrations more than 10,000 times lower than its MNTD.

In association with dCF, ddAdo reduces by one, two or three orders of magnitude the HIV multiplication at concentrations from 2 to 5 times lower than those necessary to produce the same inhibition degree as that obtained when it is used alone.

Table 5

| Anti-HIV-1 effect of ddAdo, alone or in association with desoxycoformycin. | | | |
|---|---|---|---|
| desoxycoformycin | ddAdo ($\mu$M) | | |
| | (a)ED90 | ED99 | ED99.9 |
| Absent | 4.8 | 14.3 | 23.8 |
| 50 | 0.9 | 3.8 | 6.7 |
| 5 | 1.1 | 6.2 | 11.2 |
| 0.5 | 1.3 | 7.8 | 14.2 |
| 0.05 | 2.5 | 8.0 | 14.4 |

(a)ED90, ED99, Ed99.9 (Effective dose 90, 99, 99.9): concentration of ddAdo, alone or in association, necessary to reduce by 90, 99, or 99.9% the HIV-1 multiplication in MT4 cells.

The virus titre in untreated controls was $5.5 \times 10^5$ CCID50/mL.

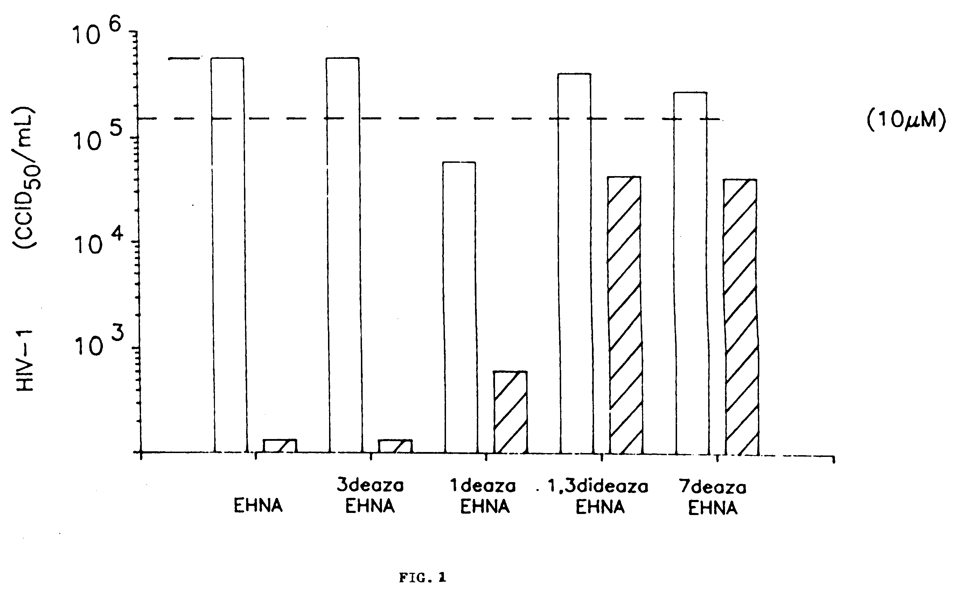

The effect of the associations ddAdo/EHNA or its deaza derivatives on the HIV-1 multiplication in MT4 cells is disclosed in fig. 1 wherein the results are reported of the enhancement of the anti-HIV-1 activity of

ddAdo (10 $\mu$M) by EHNA and its deaza derivatives, all used at the corresponding MNTD.

In this figure the horizontal hatched line represents the effect of ddAdo used alone (10 $\mu$M), the non hatched bars represent the effect of EHNA and its deaza-derivatives alone and the hatched bars represent the effect of ddAdo associated with these compounds.

As it may be noted, EHNA and 3-deaza-EHNA are the two compounds having the highest enhancing effect for ddAdo.

In order of decreasing enhancing ability follow 1-deaza-EHNA, 7-deaza-EHNA and 1,3-dideaza-EHNA.

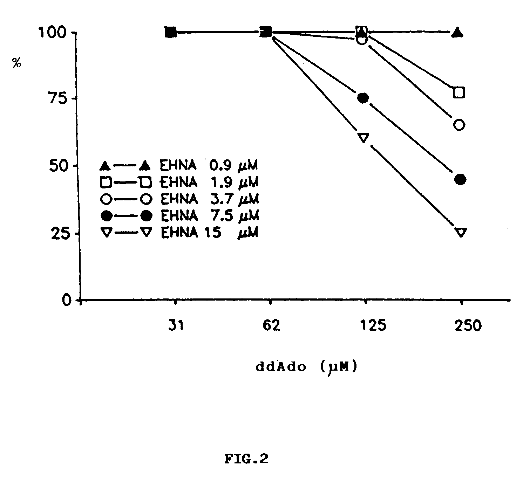

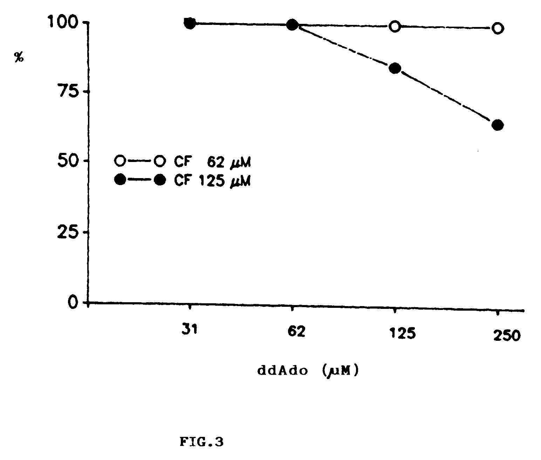

The evaluation of the toxicity enhancement of ddAdo by EHNA and Coformycin (CF) in MT4 cells is explained respectively in Fig. 2 and in Fig. 3 wherein ordinate represents the percentage of vital cells in comparison with the controls and abscissa represents the MNTD of ddAdo in $\mu$M.

As it clearly appears from fig. 2, by using a dose equal to 15 $\mu$M of EHNA the MNTD of ddAdo is reduced from >1000 $\mu$M to 60 $\mu$M in MT4 cells.

Lower concentrations of EHNA affect less and less ddAdo toxicity.

These results clearly show that the concentrations of EHNA and ddAdo able to cause cytotoxicity in association are much higher than those necessary to obtain an effective antiviral effect.

As it can be observed in Fig. 3 the MNTD of ddAdo are reduced to 60 $\mu$M only by using doses of 125 $\mu$M of CF or concentrations of two orders of magnitude higher than those necessary to enhance the ddAdo anti-HIV activity.

The above reported results allow to conclude what follows.

EHNA, 3-deaza-EHNA, CF and dCF enhance the ddAdo anti-HIV activity. This allows to use ddAdo doses up to 8-11 times lower than those necessary to obtain the same inhibition degree when ddAdo is used alone.

Also 1-deaza-EHNA, 1,3-dideaza-EHNA and 7-deaza-EHNA enhance the ddAdo anti-HIV activity, although in a lower degree.

The enhancement of the ddAdo anti-HIV activity is obtained by using concentrations of EHNA, 3-deaza-EHNA, CF and dCF much lower than the respective maximum non toxic doses.

The use of EHNA or CF in association with ddAdo, in the range of concentrations inhibiting the HIV multiplication, allows the normal cells to maintain an unaltered multiplication rate. In fact the cytotoxic effects begin to appear only by using doses of EHNA, CF and ddAdo from 10 to 100 times higher than those necessary to significantly inhibit HIV. Neither actually a toxicity of EHNA or CF mediated by a ddAdo accumulation is to be feared.

In fact, from tests carried out in parallel to those above described, it results that also in this case the cytotoxicity appears by using EHNA and CF doses from 10 to more than 100 times higher than those able to enhance ddAdo and moreover, only in the presence of ddAdo concentrations (60 $\mu$M) which are unreachable in vivo without the nucleoside administration.

With respect to the use of ddAdo alone, a first advantage in the use of ddAdo in association with EHNA, 3-deaza-EHNA, CF or dCF in AIDS therapy consists in obtaining an anti-HIV effect at very reduced ddAdo concentrations with positive effects in terms of toxicity reduction.

It is then to be considered that the evidences in favour of a correlation between high ADA levels and AIDS progression are more and more numerous, and because of the importance of this enzyme in controlling the physiological levels of ddAdo and Ado, which perform a key role in many physiological processes, the use of measured doses of EHNA or CF in association with ddAdo is suitable not only to arrest viral multiplication but also to limit the consequences deriving from high levels of ADA.

Finally, because a series of lymphoproliferative diseases are in evident correlation with the infection caused by human retroviruses such as HTLV I and II and since,as demonstrated in HIV case, the inverse transcriptase represents an optimal target for a selective inhibition operated by 2',3' didesoxynucleotides, the use of ddAdo/EHNA or CF association represents also in these cases the treatment to be elected.

In view of the foregoing it results very interesting the use of 2',3'-didesoxyadenosine in association with substances inhibiting the adenosine-deaminase for the preparation of therapeutical compositions having anti-HIV activity.

These substances inhibiting the adenosine-deaminase are preferably 9-(erythro-2-hydroxy-3-nonyl) adenine (EHNA), 3-deaza-EHNA, coformycin (CF), desoxycoformycin (dCF), 1-deaza-EHNA, 1,3 dideaza-EHNA, and 7-deaza-EHNA.

When said substance inhibiting adenosine-deaminase is 9-(erythro-2-hydroxy-3-nonyl) adenine, the molar ratio of this substance to 2',3'-didesoxyadenosine is comprised between 5:1 and 0.005:1, whereas, when said inhibiting substance of adenosine deaminase is coformycin, the molar ratio of this substance to 2',3'-didesoxyadenosine is comprised between 45:1 and 0.002:1.

Therefore the present invention relates also to these compositions, in admixture with pharmacologically acceptable diluents and excipients, and to their use in the AIDS therapy.

**SCHEME 1**

ddIno METABOLISM

ddAdo $\xrightarrow{3}$ ddAMP $\xrightarrow{9}$ $\xleftarrow{6/7}$ ddIMP, ddAMP $\xrightarrow{4}$ ddADP → ddATP

ddIno $\xrightarrow{5}$ ddIMP $\longrightarrow$ ddIDP // ddITP

Hx $\xrightarrow{8}$ ddIMP

ddGMP → ddGDP → ddGTP

ddAdo ← ddAdo

ddIno ← ddIno

ddAdo $\xrightarrow{1}$ ddIno $\xrightarrow{2}$ Hx

purine nucleoside carrier

1: Adenosine deaminase; 2: Purine nucleoside phosphorylase;
3: Deoxycytidine kinase; 4: Adenosine kinase; 5: 5'-nucleotidase;
6: Adenylosuccinate synthetase; 7: Adenylosuccinate lyase;
8: Hypoxanthine-guanine phosphorybosyltransferase
9: Adenylate deaminase.

## Claims

1. Use of 2',3'-didesoxyadenosine and of substances inhibiting adenosine-deaminase for the preparation of therapeutic compositions having enhanced anti-HIV activity.

2. The use according to claim 1, characterized in that these substances inhibiting adenosine-deaminase are 9-(erythro-2-hydroxy-3-nonyl) adenine (EHNA) 3-deaza-EHNA, coformycin (CF), desoxycoformycin

8

(dCF), 1-deaza-EHNA, 1,3-dideaza-EHNA and 7-deaza-EHNA.

3. The use according to claim 1, characterized in that when said substance inhibiting adenosine-deaminase is 9-(erythro-2-hydroxy-3-nonyl) adenine, the molar ratio of said substance to 2',3'-didesoxyadenosine is comprised between 5:1 and 0.005:1.

4. The use according to claim 1, characterized in that when the substance inhibiting the adenosine-deaminase is coformycin, the molar ratio of this substance to 2 ,3'-didesoxyadenosine is comprised between 45:1 and 0.002:1.

5. Therapeutical compositions consisting of 2',3'-didesoxy-adenosine and of substances inhibiting adenosine-deaminase in admixture with pharmacologically acceptable diluents and excipients having enhanced anti-HIV activity.

6. The compositions according to claim 5, characterized in that said substances inhibiting adenosine-deaminase are 9-(erythro-2-hydroxy-3-nonyl) adenine (EHNA), 3-deaza EHNA, coformycin (CF), desoxycoformycin (dCF), 1-deaza-EHNA, 1,3-dideaza-EHNA and 7-deaza-EHNA.

7. The compositions according to claim 5, characterized in that when said substance inhibiting adenosine-deaminase is (9-erythro-2-hydroxy-3-nonyl) adenine, the molar ratio of this substance to 2',3' didesoxyadenosine is comprised between 5:1 and 0.005:1.

8. The compositions according to claim 5 characterized in that when said the substance inhibiting adenosine-deaminase is coformycin the molar ratio of this substance to 2',3'-didesoxyadenosine is comprised between 45:1 and 0.002:1.

9. Use of therapeutical compositions comprising 2',3'-didesoxyadenosine and substances inhibiting adenosine-deaminase for AIDS therapy.

FIG. 1

FIG.2

FIG.3

EP 0 509 470 A2